# EUROPEAN PATENT SPECIFICATION

(11) **EP 2 942 042 B1**
(45) Date of publication and mention of the grant of the patent: **17.01.2018**
(21) Application number: 13883253.0
(22) Date of filing: 26.06.2013
(51) Int. Cl.: A61F 11/00, A61B 5/12, H04R 25/00

(54) **TINNITUS TREATMENT DEVICE**
TINNITUSBEHANDLUNGSVORRICHTUNG
DISPOSITIF DE TRAITEMENT D'ACOUPHÈNES

(30) Priority: 27.04.2013 CN 201310151686
(43) Date of publication of application: 11.11.2015
(73) Proprietor: Jiangsu Betterlife Medical Co., Ltd., Jiangsu 215500 (CN)
(72) Inventor: ZHAO, Bingbing, Jiangsu 215500 (CN); ZHAO, Yong David, Jiangsu 215500 (CN); ZHAO, Jennifer Jinping, Jiangsu 215500 (CN)
(74) Representative: Becker Kurig Straus
(86) International application number: PCT/CN2013/000760
(87) International publication number: WO 2014/172813

(56) References cited:
- WO-A1-2011/043678
- CN-A- 1 911 194
- CN-A- 102 647 944
- CN-U- 202 859 447
- CN-Y- 201 139 570
- US-A- 4 759 070
- US-A1- 2012 203 130

## Description

### FIELD OF THE INVENTION

The present disclosure generally relates to the technical field of medical equipments, and more particularly, to a tinnitus treatment device.

### BACKGROUND OF THE INVENTION

Tinnitus is a common clinical symptom. Tinnitus typically refers to a subjective feeling for sound that is generated in the ears or the head without any external sound source or electromagnetic stimulation, i.e., subjective tinnitus (called tinnitus or cranial tinnitus for short). In a broad sense, tinnitus also includes objective tinnitus which is generated under a corresponding sound source, e.g., a vascular or myogenic noise, etc. Tinnitus is different from acousma which is a symptom that a patient can hear a sound with specific contents (e.g., music or dialogues) without external sound source.

It is difficult to determine causes of most tinnitus, and drugs approved by Food and Drug Administration for eliminating tinnitus are currently unavailable. Patients with tinnitus caused by uncertain reasons can receive tinnitus treatment to relieve tinnitus or reduce the influence of tinnitus on the patients. Tinnitus treatment prescriptions mainly include two physical therapies, i.e., tinnitus masking and tinnitus retraining. The working principle of these two therapies is mainly to reduce central excitability and increase central inhibition by retraining or recoding the nervous system so as to bring the vicious cycle of tinnitus and unhealthy emotions to an end, help the patients adapt to tinnitus and stop or reduce perception of tinnitus, thereby achieving the therapeutic effect. Tinnitus sounds found in clinical practice are infinite and vary from person to person, from the left ear to the right ear, and even vary at different time or in different environments. The key to the tinnitus physical therapies is to accurately detect personalized tinnitus and directly convert it into tinnitus treatment prescriptions.

Conventional tinnitus treatment devices can only generate relatively simple treatment prescriptions and do not support personalized programm debugging, so the therapeutic effect is not good.
From WO 2011/043678, a tinnitus masking system is known which comprises left and right ear-level audio delivery devices for delivering a virtual spatial masking sound to both ears.

### SUMMARY OF THE INVENTION

A primary technical problem to be solved by the present disclosure is to provide a tinnitus treatment device, which can generate personalized tinnitus treatment prescriptions and perform comprehensive, optimized, balanced and targeted program debugging treatment on the left ear and the right ear.

To solve the aforesaid technical problem, a technical solution adopted by the present disclosure is to provide a tinnitus treatment device, which comprises a tinnitus diagnostic-prescriptive module, a tinnitus treatment programmer module and a tinnitus treatment instrument;
the tinnitus diagnostic-prescriptive module is configured to generate an audio pitch signal of a tinnitus diagnostic prescription of a left ear and an audio pitch signal of a tinnitus diagnostic prescription of a right ear respectively, and output the audio pitch signals of the tinnitus diagnostic prescriptions of the left ear and the right ear respectively to the tinnitus treatment programmer module;
the tinnitus treatment programmer module is configured to convert the inputted audio pitch signals of the tinnitus diagnostic prescriptions of the left ear and the right ear respectively into audio pitch signals of tinnitus treatment prescriptions of the left ear and the right ear according to a predefined formula, interact with the patient to perform comprehensive, optimized and balanced program debugging processing on the both ears, and output audio pitch signals of the processed tinnitus treatment prescriptions of the left ear and the right ear to the tinnitus treatment instrument respectively.

In a preferred embodiment of the present disclosure, the tinnitus treatment instrument includes a tinnitus treatment instrument having a hearing compensation function and a tinnitus treatment instrument not having a hearing compensation function;
the tinnitus treatment instrument having the hearing compensation function is capable of inputting, fixing and adjusting the audio pitch signals of the tinnitus treatment prescriptions of the left ear and the right ear as well as signals of hearing compensation prescriptions of the left ear and the right ear,
and the tinnitus treatment instrument not having the hearing compensation function is capable of inputting, fixing and adjusting the audio pitch signals of the tinnitus treatment prescriptions of the left ear and the right ear.

In a preferred embodiment of the present disclosure, the audio pitch signals of the tinnitus diagnostic prescriptions generated by the tinnitus diagnostic-prescriptive module include one or a combination of a pure tone, a pulse pure tone, a hybrid pure tone, a hybrid pulse pure tone, a white noise, a colored noise, a wide-band/narrow-band noise, a single warble tone, multiple hybrid warble tones, a pulse warble tone, a recorded audio signal, a preset tinnitus sound, or a natural sound. The tinnitus treatment prescription of the left ear may be the same as or different from the tinnitus treatment prescription of the right ear.

In a preferred embodiment of the present disclosure, the tinnitus treatment device further comprises a remote tinnitus diagnostic and treatment module for remotely debugging the tinnitus signals, and machine-to-machine interaction and signal transmission can be achieved by transmitting signals via a mobile phone open platform and installing a wireless transmitting module GPRS on the tinnitus treatment instrument.

In a preferred embodiment of the present disclosure, the tinnitus diagnostic-prescriptive module further comprises a tinnitus signal library unit, and when the tinnitus patient cannot be present at the site or no tinnitus symptom can be found when an intermittent tinnitus patient is being detected, tinnitus treatment audio prescriptions that are possibly similar to the tinnitus of the patient are restored or debugged according to the description of the tinnitus symptom and then downloaded into the tinnitus treatment instrument so that the patient debugs several preset prescriptions by himself to find one or more prescriptions similar to the tinnitus of the patient and then feed back the tinnitus to a tinnitus treatment specialist.

In a preferred embodiment of the present disclosure, the tinnitus diagnostic-prescriptive module comprises a fully digital audio tone signal synthesis generator for generating various audio tone signals necessary for tinnitus diagnostics and a manipulating module for manipulating the fully digital audio tone signal synthesis generator, the fully digital audio tone signal synthesis generator comprises a composite sound signal generating module, a pure tone signal generating module, a noise signal generating module and a recorded signal generating module, and the composite sound signal generating module, the pure tone signal generating module, the noise signal generating module and the recorded signal generating module generate specific audio tone signals respectively.

In a preferred embodiment of the present disclosure, a left-ear fully digital audio tone signal synthesis generator is configured to generate an audio tone signal needed by the tinnitus treatment prescription of the left ear;
a right-ear fully digital audio tone signal synthesis generator is configured to generate an audio tone signal needed by the tinnitus treatment prescription of the right ear; and
the manipulating module is configured to manipulate the left-ear fully digital audio tone signal synthesis generator and the right-ear fully digital audio tone signal synthesis generator respectively or manipulate the left-ear fully digital audio tone signal synthesis generator and the right-ear fully digital audio tone signal synthesis generator simultaneously in an interactive manner so as to optimize and adjust the audio tone signals inputted into the left ear and the right ear, stably restore the tinnitus sounds of the both ears, comprehensively evaluate the tinnitus and output tinnitus diagnostic audio tone signals.

In a preferred embodiment of the present disclosure, the left-ear fully digital audio tone signal synthesis generator and the right-ear fully digital audio tone signal synthesis generator are capable of copying data of each other so that the data are adjusted on the basis of the copying.

In a preferred embodiment of the present disclosure, the composite sound signal generating module comprises:
a multi-channel synthesis module comprising one or more independent mono signal generators, wherein each of the mono signal generators generates a sound at an arbitrarily given frequency and any arbitrarily given sound level range, the contribution degree of each of the mono signal generators in the composite sound is substantially determined by loudness of the mono signal generator at the given frequency, and the tone, the melody, the rhythm, and the loudness of the composite audio tone signal are determined by the arrangement of the mono signal generators and the contribution degrees of each of the mono signal generators, and the total loudness of the composite sound is separately adjustable;
a preset signal waveform module, being configured to adjust waveform parameters according to a preset audio signal waveform including but not limited to a straight wave, a shock wave, a ramp wave, a sawtooth wave, a trigonometric function (sine, cosine, tangent, and cotangent) wave, a trapezoidal wave, a rectangular wave or an impulse wave; and
a tinnitus signal library module, being configured to save and invoke various tinnitus digital signals according to their categories, and according to the personalized testing demand of the tinnitus patient, fine tune and invoke parameters including but not limited to the frequency, the loudness and the waveform of a tinnitus reference signal to restore and output a tinnitus sound signal satisfying the personalized diagnostic demand of the patient.

The benefits of the present disclosure are as follows: the tinnitus treatment device of the present disclosure can perform program debugging on the single ear or on the both ears simultaneously in an interactive manner; balanced debugging performed on the both ears can provide a better therapeutic effect for most tinnitus patients and optimize and stably restore the tinnitus of the both ears; and various audio tone signal synthesis modes provide more personalized tinnitus treatment prescriptions.

### BRIEF DESCRIPTION OF THE DRAWINGS

FIG. 1 is a schematic block diagram of a preferred embodiment of a tinnitus treatment device according to the present disclosure;
FIG. 2 is a schematic block diagram of a tinnitus diagnostic-prescriptive module;
FIG. 3 is a structural block diagram of a preferred embodiment of a tinnitus treatment manipulating module; and
FIG. 4 is a structural block diagram of a fully digital audio tone signal synthesis generator shown in FIG. 3.

### DETAILED DESCRIPTION OF THE INVENTION

Hereinafter, preferred embodiments of the present disclosure will be detailed with reference to the attached drawings so as to make the advantages and features of the present disclosure more readily appreciated by those skilled in the art and to define the claimed scope of the present disclosure more clearly and definitely.

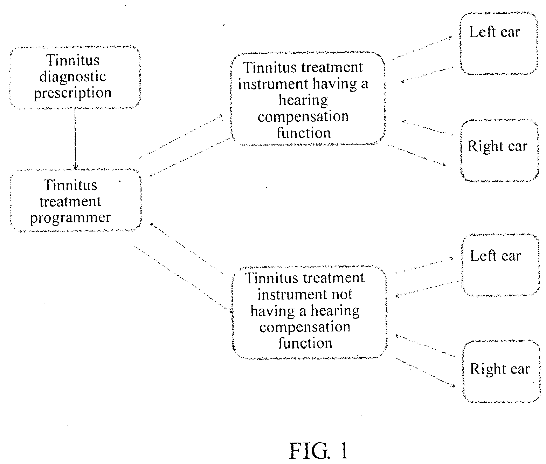

Referring to FIG. 1, an embodiment of the present disclosure provides:
a tinnitus treatment device, which comprises a tinnitus diagnostic-prescriptive module, a tinnitus treatment programmer module and a tinnitus treatment instrument.

The tinnitus diagnostic-prescriptive module is configured to generate an audio pitch signal of a tinnitus diagnostic prescription of a left ear and an audio pitch signal of a tinnitus diagnostic prescription of a right ear respectively, and output the audio pitch signals of the tinnitus diagnostic prescriptions of the left ear and the right ear respectively to the tinnitus treatment programmer module.

The tinnitus treatment programmer module is configured to convert the inputted audio pitch signals of the tinnitus diagnostic prescriptions of the left ear and the right ear respectively into audio pitch signals of tinnitus treatment prescriptions of the left ear and the right ear according to a predefined formula, and output the audio pitch signals of the tinnitus treatment prescriptions of the left ear and the right ear to the tinnitus treatment instrument respectively.

The tinnitus treatment instrument includes a tinnitus treatment instrument having a hearing compensation function and a tinnitus treatment instrument not having a hearing compensation function. The tinnitus treatment instrument having the hearing compensation function is capable of inputting, fixing and adjusting the audio signals of the tinnitus treatment prescriptions of the left ear and the right ear as well as combined inputted signals of hearing compensation prescriptions of the left ear and the right ear. The tinnitus treatment instrument not having the hearing compensation function is capable of inputting, fixing and adjusting the audio signals of the tinnitus treatment prescriptions of the left ear and the right ear.

The tinnitus treatment can perform debugging on the single ear or on the both ears simultaneously in an interactive manner, and balanced debugging performed on the both ears can provide a better therapeutic effect for most tinnitus patients and optimize and stably restore the tinnitus of the both ears. Personalized debugging may be performed after the data of the left ear and the right ear are copied for each other.

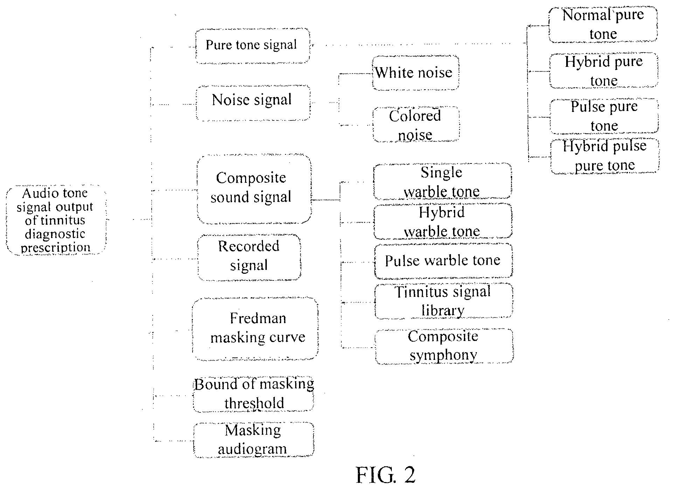

As shown in FIG. 2, the audio tone signals of the tinnitus treatment prescriptions include a pure tone, a pulse pure tone, a hybrid pure tone, a hybrid pulse pure tone, a white noise, a colored noise, a single warble tone, multiple hybrid warble tones, a pulse warble tone, a recorded audio signal, several preset tinnitus sounds and a natural sound, a Fredman masking curve, a masking audiogram, and a bound of the masking threshold. All these audio tone signals may be achieved by several audio tone signal generating modules.

The tinnitus treatment programmer module has formulas of tinnitus masking or tinnitus retraining treatment prescriptions stored therein. The tinnitus treatment programmer module comprises a Fredman masking curve unit, a masking audiogram unit, a signal data analyzing unit, and a treatment prescription determining and outputting unit.

Tinnitus signal library: tinnitus diagnostic technicians can store the diagnostic results of clinical tinnitus diagnosis of all patients into a database for later reference or medical classified statistical analysis. When the tinnitus patient cannot be present at the site or no tinnitus symptom can be found when an intermittent tinnitus patient is being detected, tinnitus treatment audio tone prescriptions that are possibly similar to the tinnitus of the patient are designed according to the description of the tinnitus symptom and then respectively downloaded into the tinnitus treatment instrument. After the patient went home, he can wear the tinnitus treatment instrument when the tinnitus occurs so as to debug several preset prescriptions by himself to find one or more prescriptions similar to the tinnitus of the patient and then feed back the tinnitus to a tinnitus treatment specialist. In this way, the scope of trials is narrowed down so as to rapidly make a definite diagnosis of tinnitus.

The tinnitus signals are remotely debugged, and machine-to-machine interaction and signal transmission are achieved by transmitting signals via a mobile phone open platform and installing a wireless transmitting module GPRS on the tinnitus treatment instrument.

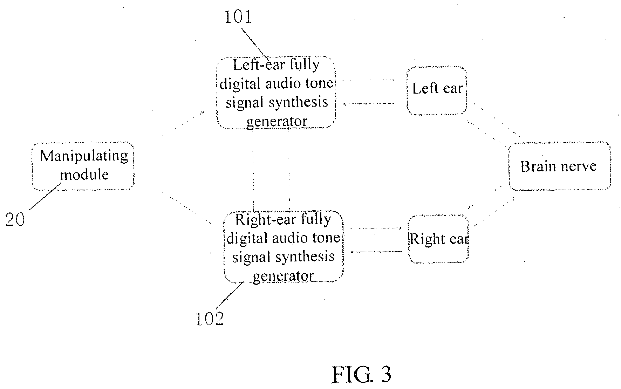

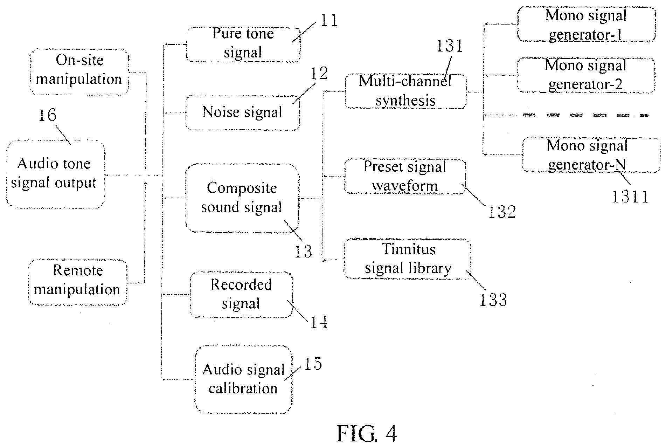

As shown in FIG. 3 and FIG. 4, the tinnitus diagnostic-prescriptive module comprises a left-ear fully digital audio tone signal synthesis generator 101, a right-ear fully digital audio tone signal synthesis generator 102 and a manipulating module 20.

The fully digital audio tone signal synthesis generator is configured to generate various audio tone signals necessary for tinnitus diagnostic tests, and the audio tone signals include a pure tone, a noise, or a composite sound, etc. Each of the left ear and the right ear is equipped with a fully digital audio tone signal synthesis generator which has an adjustable sound level range of 0 dB - 120 dB within a broadband range (e.g., 30KHz).

As shown in FIG. 2, the fully digital audio tone signal synthesis generator comprises a pure tone signal generating module 11, a noise signal generating module 12, a composite sound signal generating module 13, a recorded signal generating module 14, an audio signal calibrating module 15 and an audio signal outputting module 16.

The pure tone signal generating module 11 is configured to generate pure tone audio signals, and the pure tone is audio signals of a single frequency and intensity and may be a single continuous audio signal or disconnected audio signals.

The noise signal generating module 12 is configured to generate noise audio signals, and the noise is audio signals of different frequencies and intensities combined together randomly, and may be a single continuous audio signal or disconnected audio signals. The noise includes a white noise, a colored noise converted by a filter, and a noise of an adjustable bandwidth.

The composite sound signal generating module 13 is configured to generate composite sound audio tone signals, and the composite sound is formed by sound combination and synthesis. The composite sound signal generating module comprises a multi-channel synthesis module 131, a preset signal waveform module 132 and a tinnitus signal library module 133.

The composite sound is mainly formed in the following three ways.

The first way is to make use of multi-channel synthesis: the multi-channel synthesis module 131 comprises one or more independent mono signal generators 1311. Each of the mono signal generators can generate a sound at an arbitrarily given frequency and any arbitrarily given sound level range, e.g., 150 Hz - 8500 Hz, and 0 dB - 120 dB. The audio signals of several (e.g., two or more) independent mono signal generators are combined and synthesized according to a preset arrangement in a qualitative, positioning, and quantitative manner so as to generate the composite sound of the required tone, melody, rhythm, and loudness, just like a symphony orchestra plays a symphony according to the music score. Each channel is like a single music instrument, and the variables of the audio signal thereof include but are not limited to the frequency and the loudness. The contribution degree of each of the channels in the composite sound is substantially determined by the arrangement of the channels and the loudness of the channel at the given frequency. The total loudness of the composite sound is separately adjustable.

The second way is to make use of preset signal waveforms: such parameters as a waveform gradient, a waveform amplitude, a waveform peak extension, a waveform valley extension, a waveform period, a waveform duration are adjusted according to the preset audio signal waveforms. The horizontal axis of the waveform represents the time, and the vertical axis of the waveform represents the sound loudness. The commonly used waveforms include a straight wave, a shock wave, a ramp wave, a sawtooth wave, a trigonometric function (sine, cosine, tangent, and cotangent) wave, a trapezoidal wave, a rectangular wave, an impulse wave, a white noise, or a colored noise, etc.

The third way is to make use of a tinnitus signal library: digital signals of various tinnitus sounds that are collected after clinical medicine verification are arranged and saved according to their categories and then extracted for tinnitus diagnostics reference, and then the parameters including but not limited to the frequency and the loudness of the tinnitus sound that is extracted for reference are fine tuned according to the personalized test of the tinnitus patient so as to restore and output a tinnitus sound signal satisfying the personalized demand of the patient. The tinnitus diagnostic technician can store the diagnostic results of clinical tinnitus diagnosis of all patients into the tinnitus signal library for later reference or medical classified statistical analysis.

The recorded signal generating module 14 is configured to generate recorded audio tone signals. Hybrid sound signals satisfying the personalized demand of the patient are outputted by adjusting the frequency and the loudness of the inputted or recorded audio tone signals.

The audio signal calibrating module 15 is configured to perform sound level or sound intensity experimental calibration on the whole audio software/hardware system including a specially configured speaker headphone at a given frequency by using an internally or externally installed hearing analyzer. The audio signal calibrating module 15 can manually or automatically perform the sound level or sound intensity experimental calibration at any preset frequency and within any sound level range, e.g., 10 Hz - 8500 Hz, and 0 dB - 120 dB. The speaker headphone is placed within a vocal cavity simulating a human ear for air conductance experimental calibration.

The audio signal outputting module 16 is configured to output the debugged audio tone signals.

The manipulating module is configured to manipulate the fully digital audio tone signal synthesis generator on site or remotely.

The tinnitus treatment prescriptions outputted from the tinnitus diagnosis mainly include two physical therapies, i.e., tinnitus masking and tinnitus retraining. The working principle of these two therapies is mainly to reduce central excitability and increase central inhibition by retraining or recoding the nervous system so as to bring the vicious cycle of tinnitus and unhealthy emotions to an end, help the patients adapt to tinnitus and stop or reduce perception of tinnitus, thereby achieving the therapeutic effect. The tinnitus treatment prescriptions are the positioning for the tinnitus sound signals or expressions of the tinnitus sound signals with audio digitals, and comprise composite parameters (including the tone, the rhythm, the melody, etc) and basic parameters (including the center frequency, the bandwidth, the loudness, etc). The tinnitus treatment prescriptions may be saved as audio signal diagrams in the picture document format of pdf for printing or filing. Alternatively, time domain waveform signals may be converted into frequency domain signals by an encoder to directly output frequency domain audios in an uncompressed format or a compressed format, e.g., .wav, .mp3, .mp4, .mp5, .cad, .ram, .wma, .mid, etc. The audios may be downloaded into a tinnitus treatment instrument based on an audio signal player so that the patient can wear the tinnitus treatment instrument to listen to the audios after the audios are decoded. The tinnitus treatment prescriptions may also be directly and seamlessly inputted into a hearing aid fitting module to fit the tinnitus patients with personalized hearing aids with both the hearing compensation function and the tinnitus treatment function through program debugging.

The tinnitus diagnosis can be perform on the single ear or on the both ears simultaneously in an interactive manner to optimize and stably restore the tinnitus of the both ear, and the tinnitus diagnosis performed on the both ears can provide a better therapeutic effect for most tinnitus patients. Personalized debugging may be performed after the data of the left ear and the right ear are copied for each other. The digital audio signal synthesis generators of the left ear and the right ear can play simultaneously and alternately with the debugging.

What described above are only the embodiments of the present disclosure, but are not intended to limit the scope of the present disclosure.

## Claims

1. A tinnitus treatment device, comprising a tinnitus diagnostic-prescriptive module,
a tinnitus treatment programmer module and a tinnitus treatment instrument;
the tinnitus diagnostic-prescriptive module is configured to restore or simulate a tinnitus sound of a personalized tinnitus patient so as to directly output tinnitus diagnostic prescriptions for a single ear and/or for both ears; and
the tinnitus treatment programmer module is configured to convert the inputted tinnitus diagnostic prescriptions into tinnitus treatment prescriptions respectively according to a predefined formula, interact with the patient to perform comprehensive, optimized and balanced program debugging processing on the both ears, and output audio pitch signals of the processed tinnitus treatment prescriptions of the left ear and the right ear to the tinnitus treatment instrument respectively,
wherein said device comprises a left-ear fully digital audio tone signal synthesis generator (101), being configured to generate an audio tone signal needed by the left ear; and
a right-ear fully digital audio tone signal synthesis generator (102), being configured to generate an audio tone signal needed by the right ear,
**characterized in that** the left-ear fully digital audio tone signal synthesis generator (101) and the right-ear fully digital audio tone signal synthesis generator (102) are capable of copying data of each other so that the data are refined and adjusted on the basis of the signal copying, and
wherein said device also comprises a manipulating module (20) for manipulating the fully digital audio tone signal synthesis generators wherein the manipulating module (20) being configured to manipulate the left-ear fully digital audio tone signal synthesis generator (101) and the right-ear fully digital audio tone signal synthesis generator (102) respectively or manipulate the left-ear fully digital audio tone signal synthesis generator and the right-ear fully digital audio tone signal synthesis generator simultaneously in an interactive optimized balanced manner

2. The tinnitus treatment device of claim 1, wherein the tinnitus treatment instrument includes a tinnitus treatment instrument having a hearing compensation function and a tinnitus treatment instrument not having a hearing compensation function, the tinnitus treatment instrument having the hearing compensation function is capable of inputting, fixing and adjusting the audio pitch signals of the tinnitus treatment prescriptions of the left ear and the right ear as well as audio pitch signals of hearing compensation prescriptions of the left ear and the right ear; and the tinnitus treatment instrument not having the hearing compensation function is capable of inputting, fixing and adjusting the audio pitch signals of the tinnitus treatment prescriptions of the left ear and the right ear.

3. The tinnitus treatment device of claim 1, wherein the audio signals of the tinnitus diagnostic prescriptions generated by the tinnitus diagnostic-prescriptive module include one or a combination of a pure tone, a pulse pure tone, a hybrid pure tone, a hybrid pulse pure tone, a white noise, a colored noise, a wide-band/narrow-band noise, a single warble tone, multiple hybrid warble tones, a pulse warble tone, a recorded audio signal, a preset tinnitus sound, or a natural sound.

4. The tinnitus treatment device of claim 1, further comprising a remote tinnitus diagnostic and treatment module for remotely debugging tinnitus signals, and machine-to-machine interaction and signal transmission can be achieved by transmitting signals via a mobile phone open platform and installing a wireless transmitting module GPRS on the tinnitus treatment instrument.

5. The tinnitus treatment device of claim 1, wherein the tinnitus diagnostic-prescriptive module further comprises a tinnitus signal library unit (133), and when the tinnitus patient cannot be present at the site or no tinnitus symptom can be found when an intermittent tinnitus patient is being detected, tinnitus treatment audio tone prescriptions that are possibly similar to the tinnitus of the patient are restored or debugged according to the description of the tinnitus symptom and then downloaded into the tinnitus treatment instrument so that the patient debugs several preset prescriptions by himself to find one or more prescriptions similar to the tinnitus of the patient and then feed back the tinnitus to a tinnitus treatment specialist.

6. The tinnitus treatment device of claim 1, wherein the tinnitus diagnostic-prescriptive module comprises a fully digital audio tone signal synthesis generator for generating various audio tone signals necessary for tinnitus diagnostics, the fully digital audio tone signal synthesis generator comprises a composite sound signal generating module (13), a pure tone signal generating module (11), a noise signal generating module (12) and a recorded signal generating module (14), and the composite sound signal generating module, the pure tone signal generating module, the noise signal generating module and the recorded signal generating module (14) generate specific audio tone signals respectively.

7. The tinnitus treatment device of claim 6, wherein the composite sound signal generating module (13) comprises:
a multi-channel synthesis module (131) comprising one or more independent mono signal generators (1311), wherein each of the mono signal generators (1311) generates a sound at an arbitrarily given frequency and any arbitrarily given sound level range, the contribution degree of each of the channels in the composite sound is substantially determined by loudness of the channel at the given frequency, and the tone, the melody, the rhythm, and the loudness of the composite audio tone signal are determined by the arrangement of the mono signal generators (1311) and the contribution degrees of each of the mono signal generators, and the total loudness of the composite sound is separately adjustable;
a preset signal waveform module (132), being configured to adjust waveform parameters according to a preset audio signal waveform including but not limited to a straight wave, a shock wave, a ramp wave, a sawtooth wave, a trigonometric function wave, a trapezoidal wave, a rectangular wave or an impulse wave; and
a tinnitus signal library module (133), being configured to save and invoke tinnitus digital signals, save and invoke various tinnitus digital signals according to their categories, and according to the personalized testing demand of the tinnitus patient, fine tune and invoke such parameters as the frequency, the loudness and the waveform of a tinnitus reference signal to output a tinnitus sound signal satisfying the personalized diagnostic demand of the patient.

## Patentansprüche

1. Tinnitusbehandlungsvorrichtung, die ein Tinnitusdiagnostikpräskriptionsmodul, ein Tinnitusbehandlungsprogrammiermodul und ein Tinnitusbehandlungsinstrument aufweist; wobei das Tinnitusdiagnostikpräskriptionsmodul dazu eingerichtet ist, ein Tinnitusgeräusch eines personalisierten Tinnituspatienten wiederherzustellen oder zu simulieren, um unmittelbar Tinnitusdiagnostikpräskriptionen für ein einzelnes Ohr und/oder für beide Ohren auszugeben; und das Tinnitusbehandlungsprogrammiermodul dazu eingerichtet ist, die eingegebenen Tinnitusdiagnostikpräskriptionen jeweils gemäß einer vordefinierten Formel in Tinnitusbehandlungspräskriptionen umzuwandeln, mit dem Patienten zu interagieren, um eine umfassende, optimierte und ausgewogene Progammaustestungsverarbeitung an beiden Ohren durchzuführen, und Audioabstimmsignale der verarbeiteten Tinnitusbehandlungspräskriptionen des linken Ohrs bzw. des rechten Ohrs an das Tinnitusbehandlungsinstrument auszugeben,
wobei die Vorrichtung aufweist: einen volldigitalen Audiosignalsynthesegenerator (101) für das linke Ohr, der dazu eingerichtet ist, ein Audiosignal zu erzeugen, das für das linke Ohr erforderlich ist; und
einen volldigitalen Audiosignalsynthesegenerator (102) für das rechte Ohr, der dazu eingerichtet ist, ein Audiosignal zu erzeugen, das für das rechte Ohr erforderlich ist,
**dadurch gekennzeichnet, dass** der volldigitale Audiosignalsynthesegenerator (101) für das linke Ohr und der volldigitale Audiosignalsynthesegenerator (102) für das rechte Ohr in der Lage sind, gegenseitig Daten zu kopieren, so dass die Daten auf der Grundlage des Signalkopierens verfeinert und angepasst werden, und
wobei die Vorrichtung ferner ein Betätigungsmodul (20) zum Betätigen der volldigitalen Audiosignalsynthesegeneratoren aufweist, wobei das Betätigungsmodul (20) dafür ausgelegt ist, jeweils den volldigitalen Audiosignalsynthesegenerator (101) für das linke Ohr und den volldigitalen Audiosignalsynthesegenerator (102) für das rechte Ohr zu betätigen, oder den volldigitalen Audiosignalsynthesegenerator für das linke Ohr und den volldigitalen Audiosignalsynthesegenerator für das rechte Ohr in einer interaktiven, optimierten, ausgewogenen Weise gleichzeitig zu betätigen.

2. Tinnitusbehandlungsvorrichtung nach Anspruch 1, wobei das Tinnitusbehandlungsinstrument ein Tinnitusbehandlungsinstrument, das eine Hörkompensationsfunktion aufweist, und ein Tinnitusbehandlungsinstrument beinhaltet, das keine Hörkompensationsfunktion aufweist, wobei das Tinnitusbehandlungsinstrument, das die Hörkompensationsfunktion aufweist, in der Lage ist, die Audioabstimmsignale der Tinnitusbehandlungspräskriptionen des linken Ohrs und des rechten Ohrs sowie Audioabstimmsignale von Hörkompensationspräskriptionen des linken Ohrs und des rechten Ohrs einzugeben, festzulegen und einzustellen; und wobei das Tinnitusbehandlungsinstrument, das die Hörkompensationsfunktion nicht aufweist, in der Lage ist, die Audioabstimmsignale der Tinnitusbehandlungspräskriptionen des linken Ohrs und des rechten Ohrs einzugeben, festzulegen und einzustellen.

3. Tinnitusbehandlungsvorrichtung nach Anspruch 1, wobei die Audiosignale der Tinnitusdiagnostikpräskriptionen, die durch das Tinnitusdiagnostikpräskriptionsmodul erzeugt sind, einen reinen Ton, einen reinen Pulston, einen hybriden reinen Ton, einen hybriden reinen Pulston, ein weißes Rauschen, ein farbiges Rauschen, ein breitbandiges/schmalbandiges Rauschen, einen einzelnen Wobbelton, mehrere hybride Wobbeltöne, einen Pulswobbelton, ein aufgezeichnetes Audiosignal, einen voreingestellten Tinnitusschall, oder einen natürlichen Schall oder eine Kombination davon beinhalten.

4. Tinnitusbehandlungsvorrichtung nach Anspruch 1, ferner mit einem entfernt angeordneten Tinnitusdiagnose- und Behandlungsmodul zum Fernaustesten von Tinnitussignalen, wobei eine Interaktion und eine Signalübertragung von Maschine zu Maschine durch ein Übertragen von Signalen über eine offene Mobiltelefonplatform und ein Installieren eines drahtlosen Übertragungsmoduls GPRS an dem Tinnitusbehandlungsinstrument erreicht werden kann.

5. Tinnitusbehandlungsvorrichtung nach Anspruch 1, wobei das Tinnitusdiagnostikpräskriptionsmodul ferner eine Tinnitussignalbibliothekseinheit (133) aufweist, und wobei, wenn der Tinnituspatient an dem Ort nicht anwesend sein kann oder sich kein Tinnitussymptom beobachten lässt, wenn ein Patient mit einem zeitweise vorübergehenden Tinnitus erfasst wird, werden Tinnitusbehandlungsaudiotonpräskriptionen, die möglicherweise dem Tinnitus des Patienten ähneln, in Entsprechung zu der Beschreibung des Tinnitussymptoms wiederhergestellt oder ausgetestet und anschließend in das Tinnitusbehandlungsinstrument heruntergeladen, so dass der Patient mehrere voreingestellte Präskriptionen selbst austestet, um eine oder mehrere Präskriptionen zu finden, die dem Tinnitus des Patienten ähneln, und anschließend den Tinnitus einem Spezialisten für Tinnitusbehandlung rückzumelden.

6. Tinnitusbehandlungsvorrichtung nach Anspruch 1, wobei das Tinnitusdiagnostikpräskriptionsmodul einen volldigitalen Audiosignalsynthesegenerator aufweist, um unterschiedliche Audiosignale zu erzeugen, die zur Diagnostik eines Tinnitus erforderlich sind, wobei der volldigitale Audiosignalsynthesegenerator ein Mischschallsignalerzeugungsmodul (13), ein Reintonsignalerzeugungsmodul (11), ein Rauschsignalerzeugungsmodul (12) und ein Aufzeichnungssignalerzeugungsmodul (14) enthält, und das Mischschallsignalerzeugungsmodul, das Reintonsignalerzeugungsmodul, das Rauschsignalerzeugungsmodul und das Aufzeichnungssignalerzeugungsmodul (14) jeweils spezielle Audiosignale erzeugen.

7. Tinnitusbehandlungsvorrichtung nach Anspruch 6, wobei das Mischschallsignalerzeugungsmodul (13) aufweist:
ein Multikanalsynthesemodul (131), das einen oder mehrere unabhängige Monosignalgeneratoren (1311) aufweist, wobei jeder der Monosignalgeneratoren (1311) einen Schall bei einer beliebigen vorgegebenen Frequenz und mit einem beliebigen vorgegebenen Schallpegelbereich erzeugt, der Grad eines Beitrags jeder der Kanäle in dem Mischschall im Wesentlichen durch eine Lautheit des Kanals bei der vorgegebenen Frequenz bestimmt wird, und der Ton, die Melodie, der Rhythmus und die Lautheit des gemischten Audiosignals durch die Anordnung der Monosignalgeneratoren (1311) und die Grade des Beitrags jedes der Monosignalgeneratoren bestimmt werden, und die Gesamtlautstärke des gemischten Schalls getrennt einstellbar ist;
ein Voreinstellungssignalformmodul (132), das dazu eingerichtet ist, Wellenformparameter in Entsprechung zu einer voreingestellten Audiosignalform einzustellen, beispielsweise, jedoch ohne es darauf beschränken zu wollen, eine gerade Welle, eine Stoßwelle, eine Rampenwelle, eine Sägezahnwelle, eine Welle nach einer trigonometrischen Funktion, eine trapezförmige Welle, eine Rechteckwelle oder eine Pulswelle; und
ein Tinnitussignalbibliotheksmodul (133), das dafür ausgelegt ist, digitale Tinnitussignale zu speichern und aufzurufen, unterschiedliche digitale Tinnitussignale entsprechend ihrer Kategorien zu sichern und aufzurufen, und in Entsprechung zu der personalisierten Testanforderung des Tinnituspatienten Parameter wie die Frequenz, die Lautheit und die Wellenform eines Tinnitusreferenzsignals feinabzustimmen und aufzurufen, um ein Tinnitusschallsignal auszugeben, das die personalisierte diagnostische Anforderung des Patienten befriedigt.

## Revendications

1. Dispositif de traitement d'acouphènes, comprenant un module prescripteur de diagnostic d'acouphènes, un module de programmateur de traitement d'acouphènes et un instrument de traitement d'acouphènes, le module prescripteur de diagnostic d'acouphènes est configuré pour restituer ou simuler un son d'acouphène d'un patient souffrant d'acouphènes personnalisé de façon à émettre directement des prescriptions de diagnostic d'acouphènes pour une seule oreille et/ou les deux oreilles ; et le module de programmateur de traitement d'acouphènes est configuré pour convertir les prescriptions de diagnostic d'acouphènes entrées en prescriptions de traitement d'acouphènes respectivement selon une formule prédéfinie, interagir avec le patient pour exécuter un processus de débogage de programme complet, optimisé et équilibré sur les deux oreilles, et émettre des signaux de hauteur tonale audio des prescriptions de traitement d'acouphènes traitées de l'oreille gauche et de l'oreille droite vers l'instrument de traitement d'acouphènes respectivement,
dans lequel ledit dispositif comprend un générateur (101) de synthèse de signaux sonores audio entièrement numériques de l'oreille gauche, étant configuré pour générer un signal sonore audio dont a besoin l'oreille gauche, et
un générateur (102) de synthèse de signaux sonores audio entièrement numériques de l'oreille droite, étant configuré pour générer un signal sonore audio dont a besoin l'oreille droite,
**caractérisé en ce que** le générateur (101) de synthèse de signaux sonores audio entièrement numériques de l'oreille gauche et le générateur (102) de synthèse de signaux sonores audio entièrement numériques de l'oreille droite sont capables de copier des données l'un de l'autre de façon à ce que les données soient affinées et ajustées en se basant sur la copie de signal, et
dans lequel ledit dispositif comprend également un module manipulateur (20) pour manipuler les générateurs de synthèse de signaux sonores audio entièrement numériques dans lequel le module manipulateur (20) est configuré pour manipuler le générateur (101) de synthèse de signaux sonores audio entièrement numériques de l'oreille gauche et le générateur (102) de synthèse de signaux sonores audio entièrement numériques de l'oreille droite, respectivement, ou manipuler le générateur de synthèse de signaux sonores audio entièrement numériques de l'oreille gauche et le générateur de synthèse de signaux sonores audio entièrement numériques de l'oreille droite simultanément d'une manière équilibrée optimisée interactive.

2. Dispositif de traitement d'acouphènes selon la revendication 1, dans lequel l'instrument de traitement d'acouphènes inclut un instrument de traitement d'acouphènes ayant une fonction de compensation d'audition et un instrument de traitement d'acouphènes n'ayant pas de fonction de compensation d'audition, l'instrument de traitement d'acouphènes ayant la fonction de compensation d'audition est capable d'entrer, de fixer et d'ajuster les signaux de hauteur tonale audio des prescriptions de traitement d'acouphènes de l'oreille gauche et de l'oreille droite ainsi que les signaux de hauteur tonale audio des prescriptions de compensation d'audition de l'oreille gauche et de l'oreille droite ; et l'instrument de traitement d'acouphènes n'ayant pas de fonction de compensation d'audition est capable d'entrer, de fixer et d'ajuster les signaux de hauteur tonale audio des prescriptions de traitement d'acouphènes de l'oreille gauche et de l'oreille droite.

3. Dispositif de traitement d'acouphènes selon la revendication 1, dans lequel les signaux audio des prescriptions de diagnostic d'acouphènes générées par le module prescripteur de diagnostic d'acouphènes incluent un ou une combinaison de son pur, son pur pulsé, son pur hybride, son pur pulsé hybride, bruit blanc, bruit coloré, bruit à bande large/bande étroite, son modulé unique, sons modulés hybrides multiples, son modulé pulsé, signal audio enregistré, son d'acouphène pré-réglé ou son naturel.

4. Dispositif de traitement d'acouphènes selon la revendication 1, comprenant en outre un module de diagnostic et de traitement d'acouphènes à distance pour déboguer des signaux d'acouphènes à distance, et l'interaction machine-à-machine et la transmission de signal peuvent être obtenues par la transmission de signaux par le biais d'une plateforme ouverte de téléphone mobile et l'installation d'un module de transmission sans fil GPRS sur l'instrument de traitement d'acouphènes.

5. Dispositif de traitement d'acouphènes selon la revendication 1, dans lequel le module prescripteur de diagnostic d'acouphènes comprend en outre une unité de bibliothèque de signaux d'acouphènes (133) et lorsque le patient souffrant d'acouphènes ne peut pas être présent sur le site ou qu'aucun symptôme d'acouphènes ne peut être décelé lorsqu'un patient souffrant d'acouphènes intermittents est en cours de détection, des prescriptions de son audio de traitement d'acouphènes qui peuvent être similaires aux acouphènes du patient sont restituées ou déboguées selon la description du symptôme d'acouphènes puis téléchargées dans l'instrument de traitement d'acouphènes de façon à ce que le patient débogue plusieurs prescriptions pré-réglées par lui-même pour trouver une ou plusieurs prescriptions similaires aux acouphènes du patient et renvoyer les acouphènes à un spécialiste de traitement des acouphènes.

6. Dispositif de traitement d'acouphènes selon la revendication 1, dans lequel le module prescripteur de diagnostic d'acouphènes comprend un générateur de synthèse de signaux sonores audio entièrement numériques pour générer divers signaux sonores audio nécessaires pour les diagnostics d'acouphènes, le générateur de synthèse de signaux sonores audio entièrement numériques comprend un module générateur de signaux de sons composites (13), un module générateur de signal sonore pur (11), un module générateur de signaux de bruit (12) et un module générateur de signaux enregistrés (14) et le module générateur de signaux de sons composites, le module générateur de signal sonore pur, le module générateur de signaux de bruit et le module générateur de signaux enregistrés (14) génèrent des signaux sonores audio spécifiques respectivement.

7. Dispositif de traitement d'acouphènes selon la revendication 6, dans lequel le module générateur de signaux de sons composites (13) comprend :
un module de synthèse multi-canaux (131) comprenant un ou plusieurs générateur(s) de signal mono (1311) indépendant(s), dans lequel chacun des générateurs de signal mono (1311) génère un son sur une fréquence arbitraire donnée et n'importe quelle plage de niveaux de sons donnée, le degré de contribution de chacun des canaux dans le son composite est essentiellement déterminé par la sonie du canal à la fréquence donnée, et la tonalité, la mélodie, le rythme et la sonie du signal sonore audio composite sont déterminés par l'agencement des générateurs de signal mono (1311) et les degrés de contribution de chacun des générateurs de signal mono, et la sonie totale du son composite est réglable séparément ;
un module de forme d'onde de signal pré-réglée (132), étant configuré pour ajuster des paramètres de forme d'onde selon une forme d'onde de signal audio pré-réglée incluant mais sans s'y limiter une onde droite, une onde de choc, une onde en toit, une onde en dents de scie, une onde de fonction trigonométrique, une onde trapézoïdale, une onde rectangulaire ou une onde d'impulsion ; et
un module de bibliothèque de signaux d'acouphènes (133), étant configuré pour conserver en mémoire et appeler des signaux numériques d'acouphènes, conserver en mémoire et appeler des signaux numériques d'acouphènes divers selon leurs catégories, et selon la demande de test personnalisé du patient souffrant d'acouphènes, régler avec précision et appeler des paramètres tels que la fréquence, la sonie et la forme d'onde d'un signal de référence d'acouphènes pour émettre un signal de son d'acouphène satisfaisant la demande de diagnostic personnalisé du patient.
